# EUROPEAN PATENT APPLICATION

(11) **EP 4 295 747 A1**
(43) Date of publication of application: **27.12.2023**
(21) Application number: 23179135.1
(22) Date of filing: 14.06.2023
(51) Int. Cl.: A61B 1/00, F16B 2/22, F16B 21/06

(54) **TENSIONING DEVICE AND ENDOSCOPE ASSEMBLED THEREWITH**

(30) Priority: 22.06.2022 US 202217846926
(71) Applicant: Karl Storz Endovision, Inc., Charlton, MA 01507 (US)
(72) Inventor: Runyon, Justin, Webster, MA (US); Melanson, Jeffrey, Sturbridge, MA (US)
(74) Representative: Weidner Stern Jeschke

(57) **Abstract**

The present invention relates to a tensioning device includes a resilient body having a base and at least one prong extending generally orthogonal to the base. The base comprises a planar member, and the at least one prong has a compressive portion extending along a length of the at least one prong so as to generate a responsive force to a compression. Furthermore, the invention concerns an endoscope with a removable valve includes a tensioning device, a method of assembling an endoscope and a method of disassembling an endoscope.

## Description

This disclosure relates to a tensioning device that operates to removably retain together multiple components of an assembly, and an endoscope with components retained with the tensioning device.

Endoscopes are commonly used in the medical field for providing access to body cavities with decreased invasiveness and may include auxiliary components, such as a valve for providing suction to assist in a surgical procedure. Traditionally, such a valve is permanently affixed to a housing of an endoscope using an adhesive. Thus, separating the valve from the housing requires detaching the valve from the adhesive bond which may be a time and resource intensive procedure. Accordingly, it is desirable to have an endoscope wherein the valve may be removed without having to break an adhesive bond. Other equipment may also benefit from a means to remove components without having to break an adhesive bond.

The objective of the present invention is to improve the know state of the art.

The problem is solved by a tensioning device includes a resilient body having a base and at least one prong extending generally orthogonal to the base, the base includes a planar member, the at least one prong includes a compressive portion extending along a length of the at least one prong so as to generate a responsive force to a compression.

The tensioning device may be used to retain a removable valve to an endoscope. Therewith a tensioning device to retain multiple components together is provided. By means of the tensioning device a removable valve and/or another component can be assembled and dissembled non-destructively in and/or from a housing of an endoscope or other device, but after mounted in the housing the tensioning device keeps the valve and/or component in its position such that a user of the assembled endoscope cannot move, shift, or manipulate the removable valve until purposeful disassembly.

In one embodiment of the tensioning device, the tensioning device includes a pair of prongs, where each prong of the pair of prongs is disposed opposite of each other along a length of the base.

In another embodiment of the tensioning device, the compressive portion is an arcuate portion of the prong. Here, the arcuate portion extends a width of the at least one prong.

In an embodiment where the tensioning device includes a pair of prongs, the prongs may be symmetrical to each other. Additionally, each prong may taper towards an end of the respective prong. In these embodiments, each prong may taper with respect to a width of the respective prong. Additionally or alternatively, each prong may taper with respect to a height of the respective prong.

In another embodiment of tensioning device, the base includes a catching feature. In such an embodiment, the catching feature may be an indent.

In another embodiment of the tensioning device, each prong includes a planar inner lip portion. In these embodiments, the planar inner lip may be parallel to the planar member of the base. Here, the planar lip may also be coplanar with the planar member of the base.

In another aspect of the disclosure, the problem is solved by an endoscope, wherein the endoscope includes a housing defining an interior compartment. The housing includes a first opening closed by a cover and a second opening configured to accommodate a valve, and the valve is removably inserted into the second opening. The valve includes a mounting portion having at least one groove, the at least one groove is disposed within the interior compartment when the valve is seated within the second opening. The endoscope also includes a tensioning device, in particular a tensioning device as described above, configured to engage the at least one groove of the mounting portion. Here, the tensioning device includes a resilient body having a base and at least one prong extending generally orthogonal to the base. The base includes a planar member, and the at least one prong has a compressive portion extending along a length of the at least one prong. Here, the tensioning device engages the at least one groove of the mounting portion and the compressive portion engages an inner surface of the housing and retains the valve within the second opening under tension.

In one embodiment of the endoscope, the at least one groove has a top surface generally coplanar or contiguous with the inner surface of the housing surrounding the second opening. In such an embodiment, the at least one prong may be a pair of prongs, where each prong is disposed opposite of each other along a length of the base. Here, the pair of prongs are symmetrical to each other and the at least one groove is a pair of grooves, where the pair of grooves are symmetrical to each other.

In such an embodiment, the compressive portion may be an arcuate portion of the prongs, the arcuate portion extends a width of the prongs. Additionally, each prong may taper towards an end of the respective prong. In these embodiments, each prong may taper with respect to a width of the respective prong. Additionally or alternatively, each prong may taper with respect to a height of the respective prong.

In another embodiment of the endoscope, the base includes a catching feature. In such an aspect, the catching feature may be an indent.

In another embodiment, the endoscope further includes a stop mechanism configured to prevent the tensioning device from backing out of the at least one groove. Therefore, by means of the stop mechanism the tensioning device is retained even more securely in position.

In another aspect of the disclosure, a method of assembling an endoscope is provided. The method of assembly includes providing a housing and a cover. The housing defines an interior compartment and includes a first opening and a second opening, and the cover is configured to close the first opening. The method also includes providing a valve configured to be inserted into the second opening, the valve includes a mounting portion having at least one groove. The groove is disposed within the interior compartment when the valve is seated within the second opening. The method also includes providing a tensioning device configured to engage the groove of the mounting portion. The tensioning device includes a resilient body having a base and at least one prong extending generally orthogonal to the base. The base includes a planar member and the at least one prong has a compressive portion extending along a length of the at least one prong. The method further includes inserting the valve into the second opening so as to position the at least one groove within the interior compartment of the housing and inserting the tensioning device into the interior compartment of the housing. Here, the at least one prong is slid into the at least one groove, wherein the compressive portion is compressed against an inner surface of the housing so as to generate a responsive force and fix the valve to the housing under a tension. The method of assembly also includes placing the cover onto the first opening so as to close the housing.

In one aspect of the method of assembly, the tensioning device includes a pair of prongs. Each prong is disposed opposite of each other along a length of the base and the prongs are symmetrical to each other, and the at least one groove is a pair of grooves, and the pair of grooves are symmetrical to each other.

In another embodiment, the method of assembly further includes providing a stop mechanism within the interior compartment, the stop mechanism includes a stop. Here, the method also includes actuating the stop so as to prevent the tensioning device from backing out of the at least one groove.

In another embodiment, the method of assembly further includes providing a tool. Here, the tensioning device includes a catching feature formed on the base, and the method includes gripping the base by engagement of the tool with the catching feature and inserting the tensioning device with the tool.

In another aspect of the disclosure, a method of disassembling an endoscope is provided. The method of disassembly includes providing a housing and a cover. The housing defines an interior compartment and includes a first opening and a second opening and the cover is configured to close the first opening. The method also includes providing a valve inserted into the second opening. The valve includes a mounting portion having at least one groove, the groove is disposed within the interior compartment when the valve is seated within the second opening. The method also includes providing a tensioning device engaged with the at least one groove of the mounting portion, the tensioning device includes a resilient body having a base and at least one prong extending generally orthogonal to the base. The base includes a planar member, and the at least one prong has a compressive portion extending along a length of the at least one prong. The at least one prong is disposed within the at least one groove, where the compressive portion is compressed against an inner surface of the housing so as to generate a responsive force so as to fix the valve to the housing under a tension. The method of disassembly also includes removing the cover from the first opening so as to provide access to the interior compartment of the housing, removing the tensioning device from the at least one groove so as to free the valve from engagement with the housing, and removing the valve from the housing.

In one embodiment of the method of disassembly, the tensioning device includes a pair of prongs. Each prong is disposed opposite of each other along a length of the base and the pair of prongs are symmetrical to each other, and the at least one groove is a pair of grooves, where the pair of grooves are symmetrical to each other.

In another embodiment, the method of disassembly further includes providing a stop mechanism within the interior compartment, the stop mechanism including a stop engaged with the tensioning device and disengaging the stop so as to free the tensioning device.

In another embodiment, the method of disassembly further includes providing a tool, where the tensioning device includes a catching feature formed on the base. Here, the method includes gripping the base by engagement of the tool with the catching feature and removing the tensioning device with the tool.

The details of one or more implementations of the disclosure are set forth in the accompanying drawings and the description below. The figures show:
- FIG. 1: a top plan view of a tensioning device.
- FIG. 2: a back plan view of the tensioning device of FIG. 1.
- FIG. 3: a perspective view of the tensioning device of FIG. 1 taken from the bottom.
- Fig. 4: a side perspective view of the tensioning device of FIG. 1.
- FIG 5: a perspective view of an endoscope with a removable valve and a tensioning device.
- FIG. 6: a perspective view of the endoscope with a cover removed.
- FIG. 7: a perspective view of the endoscope with the cover and a stop mechanism removed.
- FIG. 8: a perspective view of the endoscope with the cover, the stop mechanism, and the tensioning device removed.
- FIG. 9: a perspective view of the endoscope with the cover, the stop mechanism, the tensioning device, and the valve removed.
- FIG. 10A: a close up perspective view of the endoscope shown in FIG. 9.
- FIG. 10B: an isolated view of the valve shown in FIG. 10A turned to show the inlet.
- FIG. 11: a close up perspective view of the endoscope shown in FIG. 8.
- FIG. 12: a close up perspective view of the endoscope shown in FIG. 7.
- FIG. 13: a close up front perspective view of the endoscope shown in FIG. 7.
- FIG. 14: a cross-sectional front perspective view of the endoscope taken along line 14-14 in FIG. 12.
- FIG. 15: a front perspective view of the valve of the endoscope.

Like reference symbols in the various drawings indicate like elements.

A tensioning device to removably fasten two or more components together is provided. The tensioning device includes a resilient body having a base and at least one prong. The prong extends orthogonally from the base. The prong includes a compressive portion having an arcuate surface extending along the length of the prong. The compressive portion is configured to generate a tensioned engagement between the components. Further, an endoscope having a valve removably fastened to the endoscope under tension with the tensioning device, and a method of assembling and disassembling the endoscope are provided. The endoscope includes a housing defining an interior compartment. The housing includes a first opening closed by a cover and a second opening configured to accommodate a valve, and the valve is removably inserted into the second opening. The valve includes a mounting portion having at least one groove, the at least one groove is disposed within the interior compartment when the valve is seated within the second opening. The tensioning device is configured to engage the at least one groove of the mounting portion of the valve and the interior compartment of the housing of the endoscope so as to retain the valve within the second opening under tension.

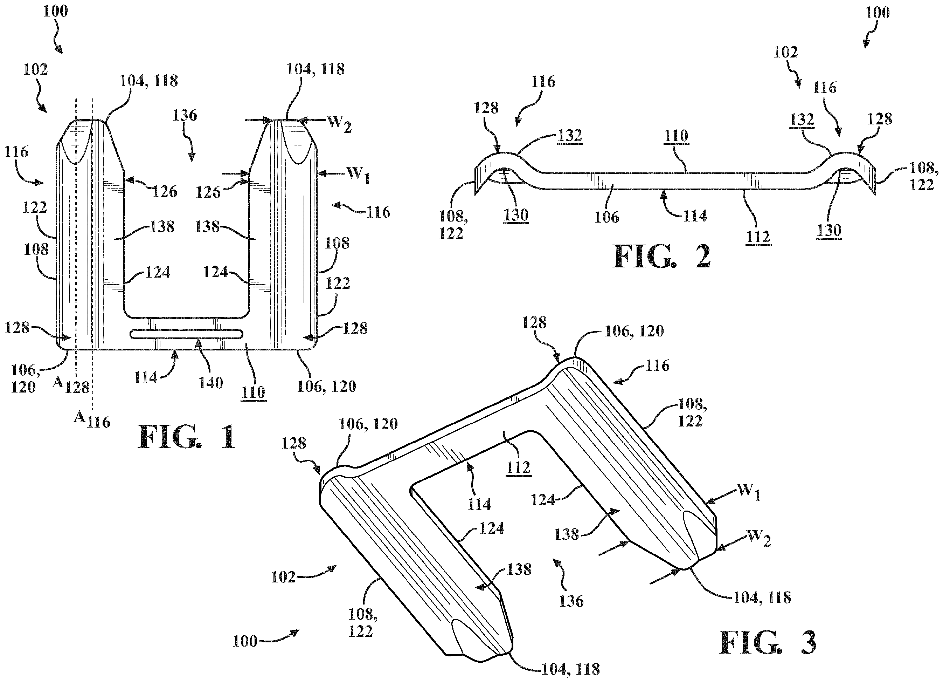

Referring to FIGS. 1-4, the tensioning device 100 includes a resilient body 102 that may be stamped and formed from durable and rigid material, such as spring steel, in a single process so as to be a unitary piece. An appropriate manufacturing jig and stamping process may be employed in the manufacture of the piece. The tensioning device 100 further includes a first end 104, a second end 106 opposite of the first end 104, opposing side surfaces 108 extending from the first end 104 to the second end 106, a top surface 110, and a bottom surface 112 opposite of the top surface 110. The tensioning device 100 further includes a base 114 and at least one prong 116. The base 114 may be primarily rectangular and planar in a central region between the side surfaces 108. The at least one prong 116 extends orthogonally from the peripheral edge of the base 114 and defines the side surfaces 108 and between the second end 106 and the first end 104.

As shown in FIG. 1, the tensioning device 100 includes a pair of prongs 116 each on a respective opposing peripheral edges of the base 114. The base 114 may further include a catching feature 140 formed in the base 114 of the tensioning device 100 and preferably between the pair of prongs 116. The catching feature 140 may be created during initial stamping or forming process of the tensioning device 100, or it may be cut into and/or added as a protruding element later in the manufacturing process. In one aspect, the catching feature 140 is a cut out segment, an indent or a protrusion configured to facilitate the engagement of a tool, such as a pair of pliers, to grip the tensioning device 100 for removal from the endoscope 10. It should be appreciated that the catching feature 140 may be shaped otherwise to facilitate the engagement of a tool, for instance, the catching feature 140 may be an aperture or a protrusion. In some embodiments, the catching feature may be specifically designed to mate with a specialized tool to facilitate the removal and/or installation of the tensioning device. Various structures may be used without deviating from the scope of the appended claims.

Each prong 116 may extend between a first prong end 118 disposed at the first end 104 of the tensioning device 100 and a second prong end 120 disposed at the second end 106 of the tensioning device 100. Each prong 116 additionally includes an outer edge 122 corresponding to the side surfaces 108 of the tensioning device 100 and an inner edge 124 extending from the base 114. A central longitudinal axis A₁₁₆ of the prong 116 may extend along a length of the prong 116 from the first prong end 118 of the prong 116 to the second prong end 120 of the prong 116. As used herein, a longitudinal direction refers to the direction extending from the first prong end 118 of the prong 116 to the second prong end 120 of the prong 116, while a lateral direction refers to the direction transverse to the longitudinal direction and extending from the outer edge 122 of the prong 116 to the inner edge 124 of the prong 116.

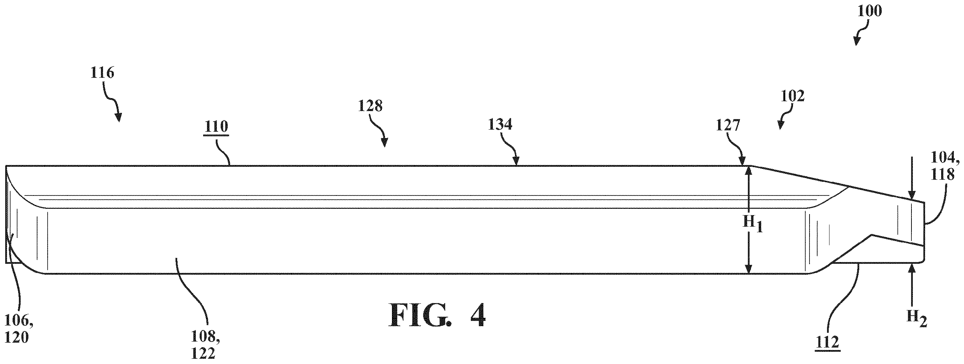

Each prong 116 may taper as it extends from the base 114 towards the first prong end 118 of the prong 116. Specifically, as shown in FIG. 1, a width of the prong 116, as defined by the lateral direction, may taper along a length of the prong 116 from a first width W1 at a first intermediate position 126 disposed between the first prong end 118 of the prong 116 and the second prong end 120 of the prong 116 to a second width W2 at the first prong end 118 of the prong 116. Similarly, as shown in FIG. 4, a height of the prong 116 may taper along the length, as defined by the longitudinal direction, of the prong 116 from a first height H1 at a second intermediate position 127, which may or may not correspond with the first intermediate position 126, between the first prong end 118 of the prong 116 and the second prong end 120 of the prong 116 to a second height H2 at the first prong end 118 of the prong 116. This tapering of the prong 116 in height and/or width can facilitate with an installation of the tensioning device.

The prong 116 may be further described as including a compressive portion 128 having an arcuate surface extending along the length of the prong 116 between the first prong end 118 of the prong 116 and the second prong end 120 of the prong 116 so as to generate a responsive force to a compression applied to the compressive portion 128. The compressive portion 128 may further be defined as including a concave inner surface 130 corresponding to the bottom surface 112 of the resilient body 102 and a convex outer surface 132 formed on the opposite side of the compressive portion 128 than the concave inner surface 130 and corresponding to the top surface 110 of the resilient body 102. The concave inner surface 130 and the convex outer surface 132 are illustratively shown in FIGS. 1-3 as having a constant radius along a central longitudinal axis A₁₂₈ of the compressive portion 128 but may be configured to have a varied radius along the central longitudinal axis A₁₂₈ of the compressive portion 128.

With continued reference to FIGS. 1-4, an illustrative example of the tensioning device 100 includes a pair of prongs 116 (i.e., two prongs 116) each disposed along a corresponding side surface 108 of the tensioning device 100 and the base 114 is integrally formed between the pair of prongs 116. In other words, each prong 116 in the pair of prongs 116 is disposed opposite of one other along the length of the base 114. Here, the pair of prongs 116 are symmetrical to each other and cooperate with the base 114 to form a cutout portion 136 sized to engage interior surfaces of a mounting portion 306 to be discussed below. As shown, each prong 116 further includes an inner lip portion 138 extending along the inner edge 124 and is coplanar with the planar portion of the base 114. The inner lip portion 138 may also be parallel, but not coplanar, with the planar portion of the base 114. It should be appreciated that while two prongs 116 are shown, the tensioning device 100 may include a single prong 116 or more than two prongs 116 without deviating from the scope of the appended claims.

In these implementations, the central longitudinal axis A₁₂₈ of the compressive portion 128 is parallel to and laterally offset from the central longitudinal axis A₁₁₆ of the prong 116. Accordingly, the compressive portion 128 laterally extends a width of the prong 116 from, or near, the outer edge 122 of the prong 116 to a position intermediate to the outer edge 122 of the prong 116 and the inner edge 124 of the prong 116. Here, the inner lip portion 138 extends between the compressive portion 128 of the prong 116 and the inner edge 124 of the prong 116. In other implementations, the width of the compressive portion 128 may define the entire width of the prong 116, resulting in no inner lip portion 138 being present. In these implementations, the central longitudinal axis A₁₂₈ of the compressive portion 128 corresponds to the central longitudinal axis A₁₁₆ of the prong 116.

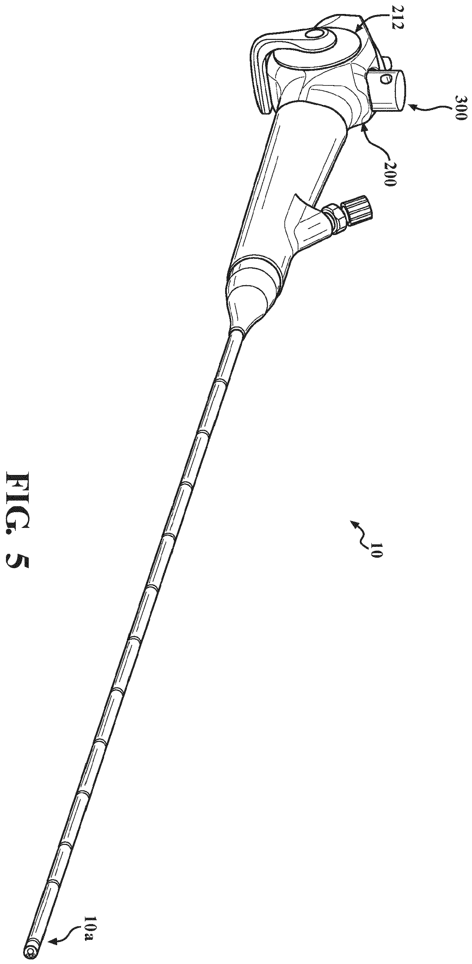

Referring now to FIGS. 5-9, in some implementations, the tensioning device 100 is used in the context of an endoscope 10. However, it should be appreciated that the tensioning device 100 may be used for other equipment where it is desired to removably couple two components together. The endoscope 10 includes a housing 200, the tensioning device 100, and a valve 300. In such a context, the housing 200 may be considered a first component and the valve 300 may be considered second component. The valve 300 is configured to provide a fluid passage into and/or out of the endoscope which may allow for the suctioning or delivery of fluids through a working end 10a of the endoscope 10. The valve 300 is shown as a single piece but may be formed of multiple parts to include an adaptor and a valve body. However, it should be appreciated that the construct of the valve 300 is not necessarily limiting to the scope of the appended claims. The valve 300 may be removably inserted in the housing 200 of the endoscope 10 and held in place axially by the tensioning device 100. The endoscope 10 may optionally include a stop mechanism 400 configured to retain the tensioning device 100 even more securely in position. As will be discussed in greater detail below, securing the valve 300 to the housing 200 using the tensioning device 100 allows the valve 300 to be assembled and disassembled to the housing 200 of the endoscope 10, and in particular, to be disassembled in a non-destructive manner.

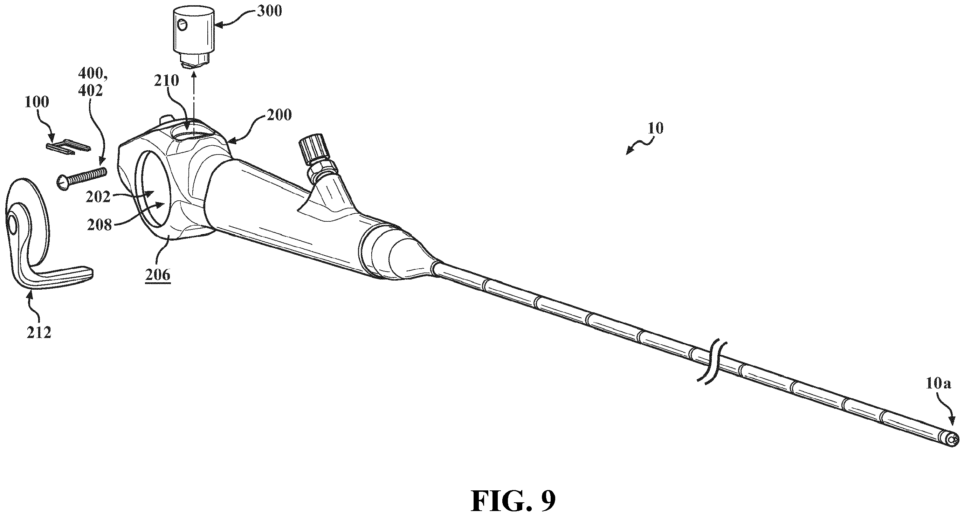

The housing 200 of the endoscope 10 defines an interior compartment 202 including an inner surface 204 and an outer surface 206. The housing 200 further includes a first opening 208 and a second opening 210 (best seen in FIG. 9). Each opening 208, 210 is formed through a thickness of the housing 200 between the inner surface 204 and the outer surface 206 and allows access to the interior compartment 202 of the housing 200. For illustrative purposes, the first opening 208 is shown as being disposed on a side of the housing 200 and the second opening 210 is shown as being disposed on a top portion of the housing 200. However, it should be appreciated that the location of the openings 208, 210 are provided for illustrative purposes and are not limiting to the scope of the appended claims. The first opening 208 may be sized to receive a cover 212 and provides direct access to the interior compartment 202, while the second opening 210 may be sized to receive the valve 300 in a sealed engagement. The inner surface 204 further includes a bracing portion 204a bounding the second opening 210 of the housing 200. When the endoscope 10 is assembled, the tensioning device 100 engages with the bracing portion 204a of the inner surface 204 to secure the valve 300 to the housing 200.

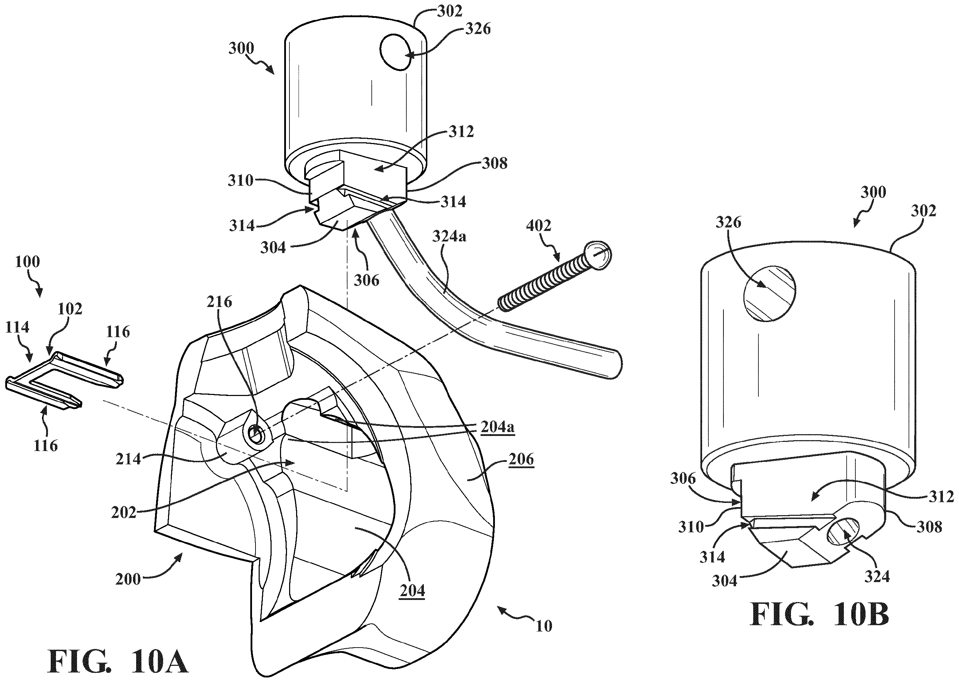

With reference to FIGS. 10A-15, the valve 300 includes a distal end 302 and a proximal end 304 formed on an opposite side of the valve 300 than the distal end 302. An inlet 324 (FIG. 10B) may formed in the proximal end 304 of the valve 300. A tube 324a may be attached to the inlet 324 and extends into the bore of the endoscope 10 to suction or provide fluids through the working end 10a of the endoscope 10. An outlet 326 is illustratively shown formed in a side of the valve 300 adjacent the distal end 302 of the valve 300. It should be appreciated that the outlet 326 may be located on other areas of the valve 300, which are exposed when the valve 300 is seated. The inlet 324 and the outlet 326 are in fluid communication to provide suction and/or fluid delivery to the endoscope 10. The valve 300 further includes a mounting portion 306 disposed at the proximal end 304 of the valve 300 and is configured to slideably engage with the tensioning device 100.

The mounting portion 306 may further be defined by a first end 308, a second end 310 formed on an opposite end of the mounting portion 306 than the first end 308, and side surfaces 312 extending between the first end 308 and the second end 310. One or more linear slot guides, such as grooves 314 are formed in the mounting portion 306 and extend along a length of side surfaces 312 of the mounting portion 306 from the first end 308 of the mounting portion 306 to the second end 310 of the mounting portion 306. In alternative embodiments, the linear slot guides may be a single groove formed circumferentially about the mounting portion 306. This circumferential groove, in some implementations, would a circularly symmetrical valve (or other element to be retained) in an axial direction, but would permit a rotational degree of freedom about the central axis of the circumferential groove.

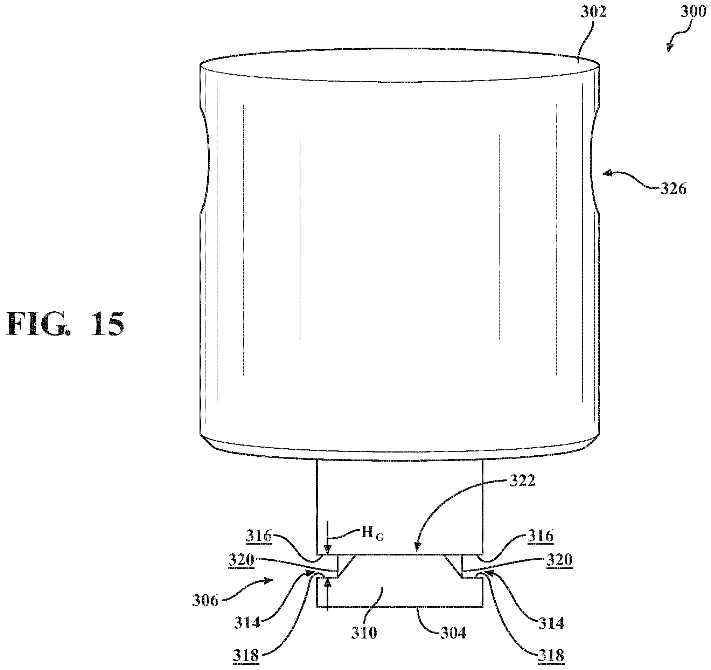

As best shown in the example of FIG. 15, the mounting portion 306 includes two grooves 314 (i.e., a pair of grooves 314), whereby each groove 314 is defined by a top surface 316, a bottom surface 318 formed on an opposite side of the groove 314 than the top surface 316, and an interior surface 320 extending between the top surface 316 of the groove 314 and the bottom surface 318 of the groove 314. The interior surfaces 320 of the grooves 314 may have a constant height "H_{G}" along a respective length of the grooves 314. A base 322 is formed between the two grooves 314, where the two grooves 314 are symmetrical about the base 322 of the mounting portion 306. It should be appreciated that while two grooves 314 are shown, the mounting portion 306 may include a single groove 314, or more than two grooves 314 without deviating from the scope of the appended claims.

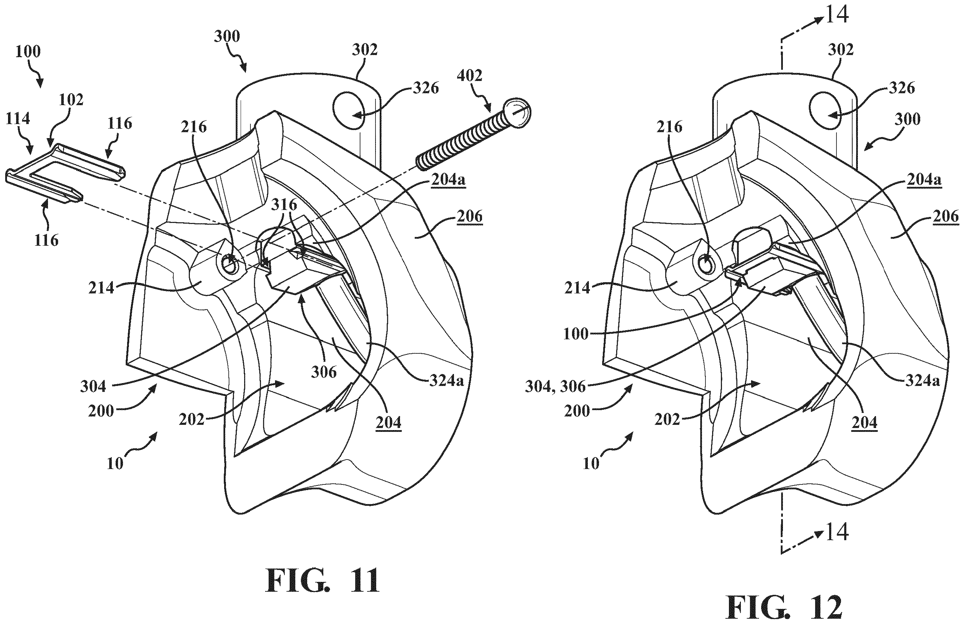

Referring now to FIGS. 10A-14, the valve 300 may be removably secured to the endoscope 10 via the tensioning device 100. As shown, the valve 300 is inserted into the second opening 210 of the housing 200 of the endoscope 10. When the valve 300 is fully seated in the second opening 210 (FIG. 11), the top surfaces 316 of the grooves 314 of the mounting portion 306 may be coplanar with the inner surface 204 of the housing 200. In this position, the mounting portion 306 extends into and is disposed within the interior compartment 202 of the housing 200. Once the valve 300 is seated, the tensioning device 100 is inserted into the mounting portion 306 of the valve 300 (FIG. 12). Here, the tensioning device 100 engages the at least one groove 314 of the mounting portion 306, where the compressive portion 128 of the tensioning device 100 engages the inner surface 204 of the housing 200 so as to retain the valve 300 within the second opening 210 under tension. Specifically, the compressive portions 128 provide a high spring force that axially locks the valve 300 into position within the housing 200 of the endoscope 10.

As shown in FIGS. 12-14, the pair of prongs 116 of the tensioning device 100 correspond to the pair of grooves 314 of the valve 300 and are configured to engage the pair of grooves 314 and engage the interior surfaces 320 of the grooves 314 at the sides of the base 322 of the mounting portion 306. When the valve 300 is seated in the second opening 210, the top surfaces 316 of the grooves 314 are generally aligned with the bracing portion 204a of the inner surface 204 of the interior compartment 202 of the housing 200.

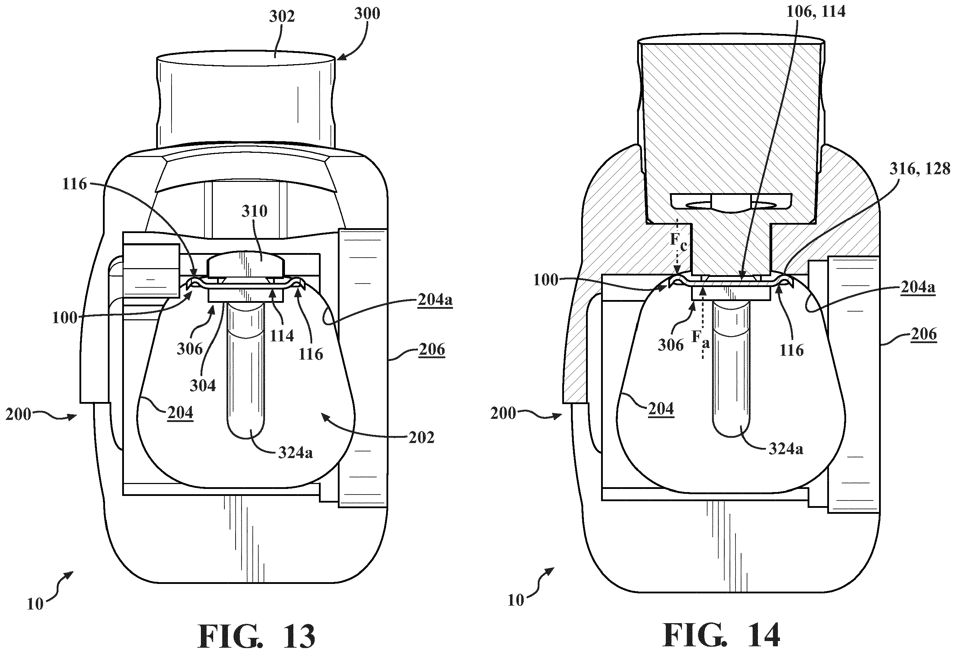

Referring to FIG. 14, the inner lip portion 138 is disposed within a respective groove 314 and the compressive portion 128 is laterally displaced from the inner lip portion 138 so as to place the convex outer surface 132 into engagement with bracing portion 204a of the inner surface 204. The engagement of the convex outer surface 132 with the bracing portion 204a of the inner surface 204 generates a compressive force F_{c}, while the bottom surface 112 of the tensioning device 100 engages with the bottom surface 318 of the grooves 314 and generates a resistive force Fₐ opposing and in response to the compressive force F_{c}. These opposing forces fix the housing 200, the tensioning device 100, and the valve 300 together under tension. The opposing forces are high enough such that a user of the assembled endoscope 10 cannot move, shift, or manipulate the removable valve 300 when the tensioning device 100 is positioned within the grooves 314 of the mounting portion 306. Advantageously, the tensioning device 100 may be inserted and removed from the valve 300 using simple tools (e.g., a flathead screwdriver, pliers, etc.,), rather than using adhesive or more complicated and destructive assembly/disassembly methods.

Optionally, the endoscope 10 includes a stop mechanism 400 disposed within the interior compartment 202 of the housing 200. The stop mechanism 400 is configured to retain the tensioning device 100 in engagement with the grooves 314 of the mounting portion 306. The stop mechanism may include the stop mount 214 disposed on the inner surface 204 of the housing 200 adjacent to the second opening 210. The stop mount 214 includes a recess 216, whereby a stop 402 is inserted into the recess 216 of the stop mount 214 and is dimensioned so as to protrude from the recess 216 when seated therein. The stop 402 is illustratively shown as a threaded screw and the recess 216 is threaded to retain the threaded screw. However, it should be appreciated that the stop 402 may be a pin or a bolt. When the stop 402 is inserted into the recess 216 (i.e., actuated), the stop 402 may engage with the second end 106 of the tensioning device 100 to prevent the tensioning device 100 from backing out of the grooves 314 of the valve 300. In these implementations, the tensioning device 100 is only accessible when the stop 402 is removed from the recess 216 of the stop mechanism 400.

Referring again to FIGS. 5-9, a method for disassembling an endoscope 10 is disclosed. The method includes providing a housing 200 defining an interior compartment 202 and including a first opening 208 and a second opening 210. The method also includes providing a cover 212 closing the first opening 208 and a valve 300 (also referred to as a valve adapter 300) inserted into the second opening 210. The valve 300 includes a mounting portion 306 having at least one groove 314. The at least one groove 314 is disposed within the interior compartment 202 when the valve 300 is seated within the second opening 210. The method also includes providing a tensioning device 100 engaged with the at least one groove 314 of the mounting portion 306. The tensioning device 100 includes a resilient body 102 having a base 114 and at least one prong 116 extending orthogonally to the base 114. The base 114 includes a planar member, and the at least one prong 116 includes a compressive portion 128 extending along a length of the at least one prong 116. When assembled, the at least one prong 116 is disposed within the at least one groove 314, where the compressive portion 128 is compressed against an inner surface 204 of the housing 200 so as to generate a responsive force F_{C} so as to fix the valve 300 to the housing 200 under tension.

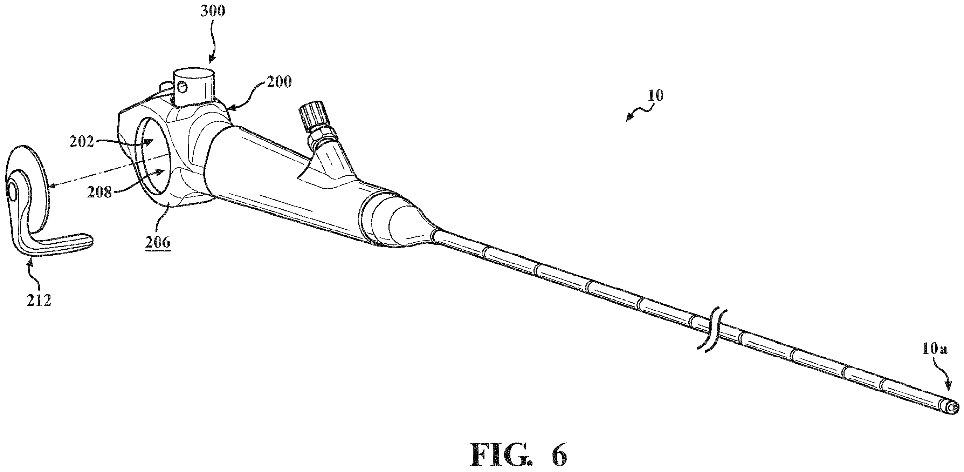

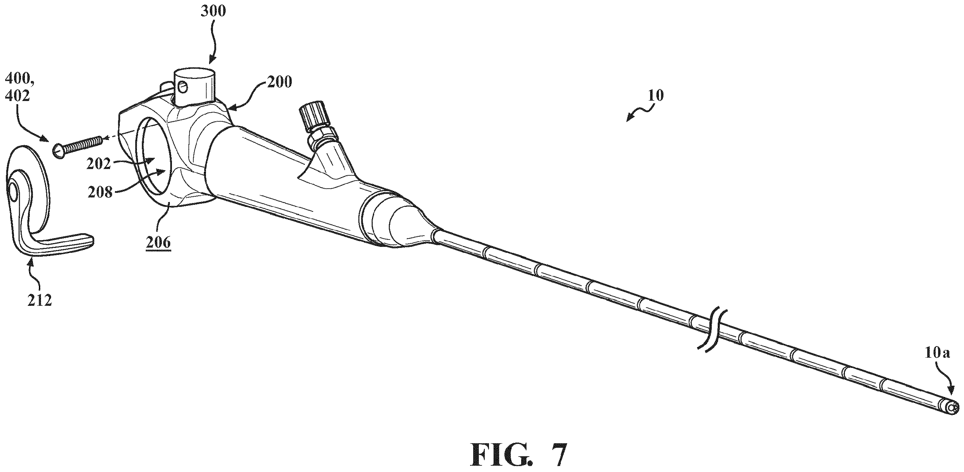

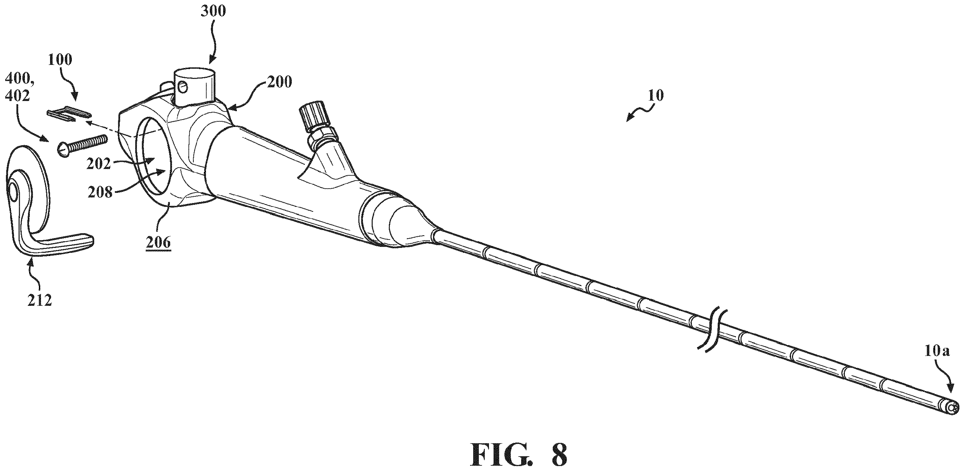

As shown in FIG. 6, the method of disassembly includes removing the cover 212 from the first opening 208 so as to provide access to the interior compartment 202 of the housing 200. At FIG. 7, the method optionally includes, in instances where a stop mechanism 400 is included, removing a stop 402 from the recess 216 of the stop mount 214 within the interior compartment 202 so as to disengage the stop 402 from the tensioning device 100 to free the tensioning device 100. As shown in FIG. 8, the method further includes removing the tensioning device 100 from the at least one groove 314 so as to free the valve 300 from the engagement with the housing 200. This may be performed by using a tool to simply pull the tensioning device 100 from the grooves 314. In embodiments where a catching feature 140 is present on the tensioning device 100, the tool may engage the catching feature to further facilitate the removal of the tensioning device. As shown in FIG. 9, the method also includes removing the valve 300 from the housing 200, by simply pulling the valve 300 from the second opening 210.

With continued reference to FIGS. 5-9, a method for assembling an endoscope 10 is disclosed. The method includes, as shown in FIG. 9, providing a housing 200 defining an interior compartment 202 including a first opening 208 and a second opening 210 and a cover 212 configured to close the first opening 208. The method also includes providing a valve 300 configured to be inserted into the second opening 210. The valve 300 includes a mounting portion 306 having at least one groove 314. The at least one groove 314 is disposed within the interior compartment 202 when the valve 300 is seated within the second opening 210. The method also includes providing a tensioning device 100 configured to engage the at least one groove 314 of the mounting portion 306. The tensioning device 100 includes a resilient body 102 having a base 114 and at least one prong 116 extending generally orthogonally to the base 114. The base 114 includes a planar member, and the at least one prong 116 includes a compressive portion 128 extending along a length of the at least one prong 116.

As shown in FIG. 8, the method of assembly includes inserting the valve 300 into the second opening 210 so as to position the at least one groove 314 within the interior compartment 202 of the housing 200. The method of assembly also includes, as shown in FIG. 7, inserting the tensioning device 100 into the interior compartment 202 of the housing 200, wherein the at least one prong 116 of the tensioning device 100 is slid into the at least one groove 314 of the valve 300. Here, the compressive portion 128 of the at least one prong 116 is compressed against an inner surface 204 of the housing 200 so as to generate a responsive force F_{C} so as to fix the valve 300 to the housing 200 under tension. The method of assembly optionally includes, as shown in FIG. 6 where a stop mechanism 400 is included, inserting a stop 402 into a recess 216 of a stop mount 214 within the interior compartment 202, providing a stop mechanism 400 within the interior compartment 202, the stop mechanism 400 including the stop 402 sized to be received by the recess 216 of the stop mount 214 in the interior compartment 202. Here, the method includes actuating the stop 402 so as to provide a back-up means of preventing the tensioning device 100 from backing out of the at least one groove 314. The method of assembly further includes, as shown in FIG. 5, placing the cover 212 onto the first opening 208 so as to close the housing 200.

A number of implementations have been described. Nevertheless, it will be understood that various modifications may be made without departing from the spirit and scope of the disclosure. It should be particularly noted that the valve or valve assembly 300 is only one of a plurality of elements or assemblies that may be fixedly retained within an endoscope or endoscopic system with the tensioning device 100. Any such assembly is but a variation of the embodiments discussed above and does not depart from the sprit and scope of the disclosure. Accordingly, other implementations are within the scope of the following claims

### Reference numerals

- 10: endoscope
- 10a: working end
- 100: tensioning device
- 102: resilient body
- 104: first end
- 106: second end
- 108: side surface
- 110: top surface
- 112: bottom surface
- 114: base
- 116: prong
- 118: first prong end
- 120: second prong end
- 122: outer edge
- 124: inner edge
- 126: first intermediate position
- 127: second intermediate position
- 128: compressive portion
- 130: concave inner surface
- 132: convex outer surface
- 136: cutout portion
- 138: inner lip portion
- 140: catching feature
- 200: housing
- 202: interior compartment
- 204: inner surface
- 204a: bracing portion
- 206: outer surface
- 208: first opening
- 210: second opening
- 212: cover
- 214: stop mount
- 216: recess
- 300: valve
- 302: distal end
- 304: proximal end
- 306: mounting portion
- 308: first end
- 310: second end
- 312: side surface
- 314: groove
- 316: surface
- 318: bottom surface
- 320: interior surface
- 322: base
- 324: inlet
- 324a: tube
- 326: outlet
- 400: stop mechanism
- 402: stop
- A₁₁₆: central longitudinal axis of the prong 116
- A₁₂₈: central longitudinal axis of the compressive portion 128
- Fₐ: resistive force
- F_{c}: compressive force
- H1: first height
- H2: second height
- H_{G}: constant height
- W1: first width
- W2: second width

## Claims

1. Tensioning device (100) for removably fastening a valve (300) to an endoscope (10) comprising:
a resilient body (102) having a base (114) and at least one prong (116) extending generally orthogonal to the base (114), the base (114) comprising a planar member, the at least one prong (116) having a compressive portion (128) extending along a length of the at least one prong (116) so as to generate a responsive force to a compression.

2. Tensioning device (100) as set forth in Claim 1, wherein the at least one prong (116) is a pair of prongs (115), each prong (116) of the pair of prongs (116) disposed opposite of each other along a length of the base (114).

3. Tensioning device (100) as set forth in Claim 1 or 2, wherein the compressive portion (128) is an arcuate portion of the at least one prong (116), the arcuate portion extending a width of the at least one prong (116).

4. Tensioning device (100) as set forth in Claim 2 or 3, wherein the pair of prongs (116) are symmetrical to each other.

5. Tensioning device (100) as set forth in one of the Claim 2-4, wherein each prong (116) of the pair of prongs (116) tapers towards an end of the respective prong (116) of the pair of prongs (116), a width of the respective prong (116) of the pair of prongs (116) and/or a height of the respective prong (116) of the pair of prongs (116).

6. Tensioning device (100) as set forth in one of the Claims 1-5, wherein the base (114) includes a catching feature (140) and/or wherein the catching feature (140) is an indent.

7. Tensioning device (100) as set forth in one of the Claims 2-6, wherein each prong (116) of the pair of prongs (116) includes a planar inner lip portion (138) and/or wherein the planar inner lip portion (138) is parallel to the planar member of the base (114) and/or wherein the planar inner lip portion (138) is coplanar with the planar member of the base (114).

8. Endoscope (10) comprising:
a housing (200) defining an interior compartment (202), the housing (200) including a first opening (208) closed by a cover (212) and a second opening (210) configured to accommodate a valve (300), the valve (300) removably inserted into the second opening (210);
the valve (300) including a mounting portion (306) having at least one groove (314), the at least one groove (314) disposed within the interior compartment (202) when the valve (300) is seated within the second opening (210); and
a tensioning device (100) configured to engage the at least one groove (314) of the mounting portion (306), comprising a tensioning device (100) according to one of the Claims 1 - 7, wherein the tensioning device (100) engages the at least one groove (314) of the mounting portion (306) and the compressive portion (128) engages an inner surface (204) of the housing (200) and retain the valve (300) within the second opening (210) under tension.

9. Endoscope (10) as set forth in Claim 8, wherein the at least one groove (314) has a top surface coplanar with the inner surface (204) of the housing (200) surrounding the second opening (210).

10. Endoscope (10) as set forth in Claim 8 or 9, wherein the at least one prong (116) is the pair of prongs (116), each prong (116) of the pair of prongs (116) disposed opposite of each other along the length of the base (114), the pair of prongs (116) are symmetrical to each other and wherein the at least one groove (314) is a pair of grooves (314), the pair of grooves (314) are symmetrical to each other.

11. Endoscope (10) as set forth in one of the Claims 8 - 10, further including a stop mechanism (400) configured to prevent the tensioning device (100) from backing out of the at least one groove (314).

12. Method of assembling an endoscope (10), the method comprising:
providing a housing (200) defining an interior compartment (202), the housing (200) including a first opening (208) and a second opening (210);
providing a cover (212) configured to close the first opening (208);
providing a valve (300) configured to be inserted into the second opening (210), the valve (300) including a mounting portion (306) having at least one groove (314), the at least one groove (314) disposed within the interior compartment (202) when the valve (300) is seated within the second opening (210);
providing a tensioning device (100) configured to engage the at least one groove (314) of the mounting portion (306), the tensioning device (100) including a resilient body (102) having a base (114) and at least one prong (116) extending generally orthogonal to the base (114), the base (114) comprising a planar member, the at least one prong (116) having a compressive portion (128) extending along a length of the at least one prong (116);
inserting the valve (300) into the second opening (210) so as to position the at least one groove (314) within the interior compartment (202) of the housing (200);
inserting the tensioning device (100) into the interior compartment (202) of the housing (200), wherein the at least one prong (116) is slid into the at least one groove (314), wherein the compressive portion (128) is compressed against an inner surface (204) of the housing (200) so as to generate a responsive force so as to fix the valve (300) to the housing (200) under a tension; and
placing the cover (212) onto the first opening (208) so as to close the housing (200).

13. Method as set forth in claim 12, further including:
providing a stop mechanism (400) within the interior compartment (202), the stop mechanism (400) including a stop (402); and
actuating the stop (402) so as to prevent the tensioning device (100) from backing out of the at least one groove (314).

14. Method of disassembling an endoscope (10), the method comprising:
providing a housing (200) defining an interior compartment (202), the housing (200) including a first opening (208) and a second opening (210);
providing a cover (212) closing the first opening (208);
providing a valve (300) inserted into the second opening (210), the valve (300) including a mounting portion (306) having at least one groove (314), the at least one groove (314) disposed within the interior compartment (202) when the valve (300) is seated within the second opening (210);
providing a tensioning device (100) engaged with the at least one groove (314) of the mounting portion (306), the tensioning device (100) including a resilient body (102) having a base (114) and at least one prong (116) extending generally orthogonal to the base (114), the base (114) comprising a planar member, the at least one prong (116) having a compressive portion (128) extending along a length of the at least one prong (116), the at least one prong (116) is disposed within the at least one groove (314), wherein the compressive portion (128) is compressed against an inner surface (204) of the housing (200) so as to generate a responsive force so as to fix the valve (300) to the housing (200) under a tension;
removing the cover (212) from the first opening (208) so as to provide access to the interior compartment (202) of the housing (200);
removing the tensioning device (100) from the at least one groove (314) so as to free the valve (300) from engagement with the housing (200); and
removing the valve (300) from the housing (200).

15. Method as set forth in claim 14, further including:
providing a stop mechanism (400) within the interior compartment (202), the stop mechanism (400) including a stop (402) engaged with the tensioning device (100); and
disengaging the stop (402) so as to free the tensioning device (100).
